# EUROPEAN PATENT SPECIFICATION

(11) **EP 3 043 644 B1**
(45) Date of publication and mention of the grant of the patent: **07.06.2023**
(21) Application number: 14777018.4
(22) Date of filing: 10.09.2014
(51) Int. Cl.: A01N 37/02, A01N 37/36, A01P 1/00, A61L 2/18

(54) **ANTIMICROBIAL COMPOSITION**
ANTIMIKROBIELLE ZUSAMMENSETZUNG
COMPOSITION ANTIMICROBIENNE

(30) Priority: 13.09.2013 EP 13184371; 20.09.2013 BE 201300621
(43) Date of publication of application: 20.07.2016
(73) Proprietor: Sopura S.A., 6180 Courcelles (BE)
(72) Inventor: BOUGARD, François, B-7100 La Louvière (BE); STACHURA, Pierre, B-7134 Binche (BE); VANHELLEPUTTE, Philippe, B-7110 Houdeing - Aimerie (BE); LONCIN, Hélène, F-75013 Paris (FR)
(74) Representative: AWA Benelux
(86) International application number: PCT/EP2014/069292
(87) International publication number: WO 2015/036433

(56) References cited:
- WO-A1-83/00163
- WO-A1-94/10837
- WO-A1-03/044145
- WO-A1-2013/059012
- WO-A2-2011/017367
- US-A1- 2010 297 316

## Description

### Background of the invention

The present invention relates to a new disinfectant composition, especially adapted for cleaning plant equipment in food industry, and in particular in brewery industry, as well as to a method for sanitizing a solid support surface by the use of this composition.

### State of the Art

For ensuring both product quality and health security of the consumer, it is a required practise in food and beverage industries to regularly submit the equipment to a strict cleaning so as to remove all residuals and contaminants (organic matters, fats, proteins,...) susceptible to lead to a detrimental development of microorganisms (bacteria and yeasts); it is also required to reduce microbial population on the equipment to very low levels; e.g. to safe levels as established by public regulations.

The main difficulty in cleaning surfaces in food and beverage industries such as pipes and tanks, lies on the fact that the equipment is mostly not disassembled before cleaning and that cleaning has thus to be performed in place.

Cleaning in place (CIP) of such surfaces usually consists in applying on these solid supports, at the end of a processing cycle, a first composition especially dedicated to the cleaning of the surfaces as such, rinsing with water the surfaces, then applying on these rinsed surfaces a second composition dedicated to their antimicrobial treatment. Finally, the surfaces are rinsed once again with water before starting the next processing cycle. The cleaning process is usually performed at a temperature of about 10°C for a relatively long period of more than 1 hour (generally from about 1 to about 2 hours), independently from the rinsing time. At the end of the total cleaning in place process, a population reduction of at least 99.999% (a five-log reduction) of undesired microorganisms is reached.

Different compositions using different components having among others detergent properties have already been proposed. For example, it has been proposed in WO 94/10837 to use solutions comprising an aliphatic C₆-C₁₅ fatty acid such as nonanoic acid and/or decanoic acid, a strong acid such as sulfuric acid, and minor amounts of a weak acid such as propionic acid, butyric acid or valeric acid.

EP 1 003 371 B1 discloses a bactericidal and yeast-killing disinfecting composition comprising a monocarboxylic acid with a saturated or unsaturated, straight or branched-chain alkyl radical comprising from 6 to 12 carbon atoms or a mixture of such carboxylic acids, a strong inorganic acid such as sulphuric acid, an organic acid such as acetic acid and/or glycolic acid, one or more anionic surfactants such as a surface-active sulfonate or a surface-active ether carboxylic acid, and a solubilizing agent such as butyl glycol or butyl diglycol.

US 2010/297316 discloses an antimicrobial composition comprising a mixture of octanoïc acid and lactic acid.

WO 2013/059012 discloses an antimicrobial composition comprising a mixture of nonanoic acid and propionic acid.

WO 03/044145 discloses an antimicrobial composition comprising a C₅-C₁₄ fatty acid (preferably nonanoic acid and/or decanoic acid) combined with a strong acid (like phosphoric acid or nitric acid) and acetic acid.

WO 2011/017367 discloses an antimicrobial composition comprising nonanoic acid and propionic acid.

WO 83/00163 discloses an antimicrobial composition comprising a C₆-C₁₄ fatty acid.

US 7,943,565 discloses an acidic sanitizing and/or cleaning composition capable of being diluted and comprising at least one C₁-C₄ hydroxy alkyl carboxylic acid or salt thereof, and namely an alpha (or beta) hydroxyl alkyl carboxylic acid such as lactic acid, at least one C₅-C₁₈ alkyl monocarboxylic acid or salt thereof, such as octanoïc acid, at least one dicarboxylic acid such as tartaric acid, at least one acid such as sulphuric acid, at least one solubilizer selected for example from alkyl sulfonates and alkylaryl sulfonates, at least one diluent and optionally at least one detergent.

Nowadays, there is a real industrial pressure for providing more and more performing compositions in terms of cleaning and sanitizing properties, which should be also less corrosive, more environmentally friendly, and less time consuming.

Nevertheless, the man skilled in the art aiming at providing a satisfying composition has to solve together different and complex technical problems.

The composition should be homogenous, stable and adapted for storage, for instance storage at room temperature. The composition should also be homogenous, stable and adapted for usage at low temperatures.
enough so as to reduce logistic constraints, which means that the concentrated composition must also be stable and not undergo phase separation at low or at room temperature. Such concentrated composition should also be easy to dilute in water.

The antimicrobial action of the composition should be such that it can reduce the population of microorganisms by five logs at 3°C in less than 20 minutes.

Moreover, the composition has to be efficient on a wide range of microorganisms, that is not only on both gram (+) and gram (-) bacteria, but also on yeasts such as Saccharomyces and Candida.

In addition, the composition has to foam as less as possible, as foaming may lead to an adsorption on surfaces and thereby interfere with the rinsing efficiency. Foaming may also cause damages to the equipment.

The composition should also be easy to recycle for a next use.

Finally, it is advantageous that the concentration of the solution be followed and determined continuously by electrical conductivity measurements.

### Summary of the invention

The present invention relates to a water-dilutable antimicrobial composition comprising: a first acid being selected from the group consisting of octanoic acid, nonanoic acid and decanoic acid or a mixture thereof, and a second acid being a mixture of propionic acid and lactic acid, wherein the said composition comprises at least 0.1% (preferably at least 1 %, more preferably at least 5% and possibly up to 10%) of this first acid (weight first acid:weight composition), wherein the weight ratio between this second acid and this first acid is of at least 8 (weight second acid:weight first acid) and wherein the pH of this composition is less than 5.0, and rein the said composition comprises more than 20 wt % of lactic acid (weight lactic acid weight of composition) and comprises more than 20 wt % of propionic acid weight propionic acid: weight of said composition).

Preferably the second acid of this water-dilutable composition comprises both lactic acid and propionic acid in a weight ratio comprised between (about) 0.1 and (about) 10 (weight lactic acid:weight propionic acid), still more preferably between (about) 1 and (about) 5 or even more preferably between (about) 2.0 (or about 2.5) and (about) 4 (or 3.0).

Preferably the first acid of this water dilutable composition is octanoïc acid,

Advantageously, this water-dilutable composition further comprises a third acid, preferably selected from the group consisting of nitric acid, phosphoric acid, sulphuric acid, chloric acid (i.e. strong mineral acids), acetic acid, citric acid, tartaric acid and mixture thereof (preferably sulphuric acid, possibly in addition to another third acid such as citric acid and/or acetic acid).

Preferably, the first acid, the second acid and possibly the third acid are present in this water-dilutable composition in a weight ratio comprised between 50% and 99% (weight of the sum of all these first, second and possibly third acids: weight of the said composition), more preferably between about 75% and about 90%, still more preferably between about 80% and about 85%.

Preferably, more than 1%, more preferably more than 5% of the first acid (i.e. octanoic acid, nonanoic acid, decanoic acid and mixtures thereof, is present in this water-dilutable composition (weight first fatty acid:weight composition).

Advantageously, this water-dilutable composition is detergent-free.

A closely related aspect of the present invention is thus a water-dilutable antimicrobial composition comprising at least 0.1 wt % (preferably between (about) 1 wt % and (about) 10 wt %, more preferably between (about) 2 wt % and (about) 5.5 wt % or between (about) 3 wt % and (about) 5 wt %) of a C₆-C₁₂ fatty acid (weight fatty acid:weight composition) selected from the group consisting of octanoic acid, nonanoic acid and decanoic acid, and being detergent free.

Another related aspect of the present invention is an antimicrobial composition (a water-diluted antimicrobial composition) comprising this water-dilutable composition.

Still another related aspect of the present invention is the non-therapeutic use of the water-dilutable composition of the present invention for sanitizing a (hard) surface (of a solid support, such as glass, and in particular a glass bottle), or a method of sanitizing a (hard) surface (of a solid support, such as glass, and in particular a glass bottle) by adding to it the water-dilutable composition of the invention.

Still another related aspect of the present invention is the non-therapeutic use of a composition comprising a first acid being selected from the group consisting of octanoic acid, nonanoic acid and decanoic acid and mixtures thereof, propionic acid and lactic acid for sanitizing a (hard) surface (of a solid support, such as bottle),

Another related aspect of the present invention is a non-therapeutic method for sanitizing a (hard) surface (of a solid support, such as a bottle) comprising the step of adding (to this surface) a composition comprising a first acid and a second acid being a mixture of propionic acid and lactic acid, wherein this composition comprises at least 0.01 % of this first acid (weight first acid:weight composition) and wherein the weight ratio of this second acid and this first acid is of at least 8, this first acid being selected from the group consisting of octanoic acid, nonanoïc acid and decanoic acid and mixtures thereof.

Advantageously, this non-therapeutic use or non-therapeutic method does not comprise any water rinsing and/or washing step after the addition of this composition comprising this first acid and this second acid. The present invention will be further described in the following description without limiting the invention scope

### Detailed description of the invention

Table 1 shows the antimicrobial effect of the compositions of the present invention and of a composition not comprising the second acid.

Although monocarboxylic fatty acids having a size of 6 to 12 carbon atoms (i.e. about 8 carbon atom), such as octanoic acid, have a poor solubility in water, especially at temperatures of (about) 0°C to (about) 10°C, the inventors have developed a (water-containing) sanitizing composition comprising them at high concentration, and such composition can be diluted with water.

The inventors have further found that the addition of a mixture of lactic acid and of propionic acid has allowed to avoid the addition of artificial detergents (or to reduce their amounts, or to replace them by food-compatible detergents i.e. detergents that are not deleterious to food and that do not affect the health of the consumer), while still allowing monocarboxylic fatty acids having a size of about 8 carbon atoms to remain soluble and/or not forming fatty films on the sanitized surfaces.

Such sanitizing composition can be in a concentrated form, as having a low freezing point below 5°C; in fact, advantageously, the concentrated composition of the present invention is stable and homogenous at temperatures between (about) 2°C and (about) 40°C.
In such concentrated compositions, the amount of the monocarboxylic fatty acids having a size of 6 to 12 carbon atoms is advantageously above 1 % (w fatty acid: w composition), preferably above 5%, even possibly of above 6%, 7%, 8% and up to 10%. The pH of such concentrated composition is below 5 (preferably between (about) 1.0 and (about) 4.5).

On the other hand, the composition in the diluted form (in the dilution for use in sanitizing; e.g. with monocarboxylic fatty acids having a size of about 8 carbons below 1 wt %, possibly below 0.1 wt % and even possibly at concentrations between (about) 0.01% (or 0.02) and (about) 0.05%) is also stable and homogenous at temperatures from 2°C to 40°C.

Conversely, despite the fact that the presence of organic acids, such as butyric acid, lactic acid and/or propionic acid and/or octanoic acid and/or of nonanoïc acid, are believed to be detrimental for food quality (unpleasant odour, skin-irritating properties), the inventors have found that sanitizing compositions based on such acids do not require supplementary rinsing steps, even when these compositions are used for cleaning surfaces in the food or beverage (beer, wine, oil, dairy products,...) industry or are applied in direct contact onto solid food.

Therefore, a first object of the present invention is related to a composition comprising a first acid selected from the group consisting of octanoic acid, nonanoic acid and decanoic acid, an acid (a second acid) being a mixture of lactic acid and propionic acid and possibly another (third) acid being a strong mineral acid and/or citric acid and/or acetic acid and/or tartaric acid.

Advantageously, this composition is antimicrobial (and/or it is a sanitizing composition).

In the context of the present invention, the term "antimicrobial" or "microbiologically effective" refers to the possibility to reduce the amount of a (living) given (contaminant) microorganism by at least 100 000 fold (5 logs) in less than 20 minutes at 3°C. Preferably, the (living and/or contaminant) microorganism is present on a surface. More preferably, "antimicrobial" or "microbiologically effective" refers to the capacity to simultaneously reduce (by at least 5 logs) the amount of several microorganisms, for instance of bacteria (Gram (+) and/or Gram (-)) and of (several strains of) yeast (e.g. Saccharomyces sp., Candida Sp., Lactobacillus Sp.,...).

Advantageously, this composition is water-dilutable.

Advantageously, the water-dilutable (antimicrobial) composition of the present invention can be diluted (e.g. about 100-fold) and still keeps all its cleaning properties, as well as the antimicrobial function.

In the context of the present invention, the term "water-dilutable" refers to the fact that the composition, preferably the antimicrobial composition, can be used after a dilution with (sterile and/or soft and/or distilled and/or ozone-treated) water, such as a 10-fold, a 50-fold, a 100-fold or even a 200-fold dilution with water.

In the context of the present invention, unless explicitly stated otherwise, the percentages are weight percent (for instance weight of a given acid : weight of the composition or weight ratios between given acids).

Preferably, the second acid of the (antimicrobial and/or water dilutable) composition of the present invention is a mixture of lactic acid and propionic acid, wherein preferably lactic acid represents at least 25 wt % of said mixture, more preferably at least 50 wt %, still more preferably at least 80 wt %).

The (antimicrobial and/or water dilutable) composition according to the present invention comprises both lactic acid and propionic acid, in addition to a first acid and possibly to another (third) acid such as a strong mineral acid, citric acid, tartaric acid or acetic acid, and possibly to another C₂-C₆ fatty acid substituted by one hydroxyl group at alpha or beta position and/or butyric acid and/or valeric acid.

Advantageously, the first acid comprises (or consists (essentially) of) octanoic acid.

Preferably, the first acid (octanoic acid) is present in a microbiologically effective amount (for instance in an amount of between (about) 0.01 wt % and (about) 0.1 wt % (preferably at least (about) 0.02%) in the diluted composition).

In other words, the first acid (octanoic acid) of the water-dilutable antimicrobial composition is advantageously present in an amount between (about) 1 wt % and (about) 10 wt % (weight of the first acid:weight of the water-dilutable composition), preferably between (about) 2 wt % and (about) 8 wt %, more preferably between (about) 4 wt % and (about) 6 wt %, still more preferably between (about) 5 wt % and (about) 5.5 wt %.

Advantageously, the (antimicrobial and/or water dilutable) composition comprises a strong mineral acid, selected from the group consisting of nitric acid, phosphoric acid, chlorhydric acid and sulphuric acid, preferably the strong mineral acid is sulphuric acid.

Alternatively or in addition to the strong mineral acid, the (antimicrobial and/or water dilutable) composition (further) comprises acetic acid and/or citric acid and/or tartaric acid.

Preferably all the (first, second and, possibly third) acids are present in the water-dilutable antimicrobial composition in a weight ratio comprised between 55% and 99%, more preferably between (about) 75% and (about) 90%, still more preferably between (about) 80% and (about) 85% (water representing more than 50 wt% (or even almost all) of the remaining part).

Preferably, the pH of the (antimicrobial and/or water dilutable) composition of the present invention is comprised between (about) 1 and (about) 5, even more preferably between (about) 3 and (about) 4.5.

Preferably, the (antimicrobial and/or water dilutable) composition of the present invention is detergent-free.

In the context of the present invention, the term "detergent-free" refers to the absence or to the reduction (i.e. less than 5 wt % in the water-dilutable composition, preferably less than 1 wt %, more preferably less than 0.1 wt %, still more preferably less than 0.01 wt % or even less than 0.001 wt %) of artificial detergents and/or of artificial emulsifiers and/or of artificial antifoaming agents and/or of detergent being not considered as safe for food application.

In other words, in the "detergent-free" composition (almost) no artificial detergent is added (yet some naturally-occurring compounds having amphiphilic and/or emulsifying and/or detergent properties can be present, including (less preferably food-grade) emulsifiers, for instance in the compositions according to the invention comprising low amounts of propionic acid and/or of lactic acid).

The term "food-grade emulsifiers" preferably refers emulsifiers labelled as "E Xxxx", such as to E 1518 (Triacetin), E 322 (Lecithins), E 431 (Polyoxyethylene (40) stearate), E 432 (Polyoxyethylene sorbitan monolaurate; i.e. Polysorbate 20), E 433 (Polyoxyethylene sorbitan monooleate; i.e. Polysorbate 80), E 434 (Polyoxyethylene sorbitan monopalmitate; i.e. Polysorbate 40), E 435 (Polyoxyethylene sorbitan monostearate; i.e. Polysorbate 60), E 436 (Polyoxyethylene sorbitan tristearate; i.e. Polysorbate 65), E 442 (Ammonium phosphatides), E 471 (Mono- and diglycerides of fatty acids), E 472a (Acetic acid esters of mono- and diglycerides of fatty acids), E 472b (Lactic acid esters of mono- and diglycerides of fatty acids), E 472c (Citric acid esters of mono- and diglycerides of fatty acids), E 472e (Mono- and diacetyl tartaric acid esters of mono- and diglycerides of fatty acids), E 472f (Mixed acetic and tartaric acid esters of mono- and diglycerides of fatty acids), E 473 (Sucrose esters of fatty acids), E 474 (Sucroglycerides), E 475 (Polyglycerol esters of fatty acids), E 476 (Polyglycerol polyricinoleate), E 477 (Propane-1 ,2-diol esters of fatty acids), E 491 (Sorbitan monostearate), E 492 (Sorbitan tristearate), E 493 (Sorbitan monolaurate), E 494 (Sorbitan monooleate) and E 495 (Sorbitan monopalmitate).

Preferably, the first acid (such as octanoic acid) of the (antimicrobial and/or water dilutable) composition of the present invention is comprised between (about) 0.1 wt % and (about) 20 wt % (weight of the first acid (octanoic acid): weight of the composition), preferably between (about) 1 wt % and (about) 10 wt %, more preferably between (about) 4 wt % and (about) 6 wt %, still more preferably between (about) 5 wt % and (about) 5.5 wt %.

Preferably, the mixture of lactic acid and propionic acid: first acid (such as octanoic acid) weight ratio of the (antimicrobial and/or water dilutable) composition of the present invention is comprised between about 5 and about 20, about 10 and about 16 or about 8 and about 10.

Preferably, the (antimicrobial and/or water dilutable) composition of the present invention comprises between (about) 10 wt % and (about) 80 wt % of the lactic acid/propionic acid mixture, preferably between (about) 20 wt % and (about) 70 wt %, more preferably between (about) 30 wt % and (about) 60 wt %, still more preferably between (about) 45 wt % and (about) 55 wt %.

Preferably, the (antimicrobial and/or water dilutable) composition of the present invention comprises between (about) 5 wt % and (about) 80 wt %, more preferably between (about) 10 wt % and (about) 25 wt %, still more preferably between (about) 15 wt % and (about) 20 wt % of a mixture of propionic acid and lactic acid.

Preferably, the (antimicrobial and/or water dilutable) composition of the present invention comprises between (about) 1 wt % and (about) 20 wt % of a strong mineral acid (such as sulphuric acid), preferably between 2 wt % and 15 wt %, still more preferably between (about) 4 wt % and (about) 10 wt %.

Possibly, the (antimicrobial and/or water dilutable) composition of the present invention comprises between (about) 1 wt % and (about) 20 wt % of citric acid and/or of acetic acid and/or of tartaric acid, preferably between (about) 2 wt % and (about) 15 wt %, still more preferably between (about) 4 wt % and (about) 10 wt %.

Advantageously, the weight ratio between the second acid (the sum of propionic acid and of lactic acid) and the first acid (the sum of the first acids; preferably octanoic acid) is of at least 5, preferably of at least 8, even more preferably comprised between (about) 8 and (about) 20, still more preferably between (about) 9 and (about) 16, even between (about) 10 and (about) 13, still more preferably between (about) 11 and (about) 12.

A related aspect of the present invention is a (diluted) antimicrobial composition comprising between (about) 0.1 wt % and (about) 5 wt %, more preferably between (about) 0.25 wt % and (about) 2 wt %, still more preferably between (about) 0.5 wt % (or 0.75 wt %) and (about) 1 wt % of the dilutable composition of the present invention (and thus between (about) 95 wt % and (about) 99.9 wt % of (distilled and/or soft and/or sterile and/or ozone-treated) water).

Another related aspect of the present invention is the use of the (diluted) composition of the present invention for surface cleaning and/or sanitizing.

For instance, a (diluted) composition comprising a first acid and a mixture of propionic acid and lactic acid is used for surface cleaning and/or sanitizing (preferably this (diluted) composition comprises between (about) 0.01 wt % and (about) 0.1 wt % (e.g. at least 0.02 wt %) of the first acid and/or the weight ratio between the second acid (lactic acid + propionic acid) and the first acid (preferably octanoïc acid) is comprised in this composition between (about) 8 and (about) 20, preferably between (about) 9 and (about) 16, more preferably between (about) 10 and (about) 13, still more preferably between (about) 11 and (about) 12 and/or the weight ratio between lactic acid and this C₆-C₁₂ fatty acid is comprised in this composition between (about) 8 and (about) 10 and/or the weight ratio between lactic acid and propionic acid is comprised in this composition between (about) 1 and (about) 5, preferably between (about) 2 and (about) 4); preferably, this composition is used at a temperature between (about) 0°C and (about) 25°C, more preferably between (about) 2°C and (about) 15°C, still more preferably between (about) 3°C and (about) 10°C (or even between (about) 4°C and (about) 8°C).

Another related aspect of the present invention is a method for sanitizing a solid support (hard) surface used or to be used in the food or beverage industry (e.g. beer, wine, oil, dairy,...), comprising the step of adding a composition comprising a mixture of lactic acid and propionic acid , a first acid (such as octanoïc acid) and, possibly an acid (a third acid) selected from the group consisting of nitric acid, phosphoric acid, sulphuric acid, acetic acid, citric acid, tartaric acid chloric acid and mixture thereof (i.e. the composition according to the present invention); preferably, this method is carried out at a temperature between (about) 0°C and (about) 25°C, more preferably between (about) 2°C and (about) 15°C, still more preferably between (about) 3°C and (about) 10°C (or even between (about) 4°C and (about) 8°C).

Advantageously, the method of the present invention does not comprises a step of water rinsing and/or washing after the addition of the composition of the present invention.

Another related aspect of the present invention is the use of the (diluted) composition of the present invention (preferably comprising octanoïc acid, lactic acid, propionic acid and optionally another acid, and/or wherein this (diluted) composition comprises between about 0.01 wt % and (about) 0.1 wt % (e.g. at least 0.02 wt %) of the first acid and/or wherein the weight ratio between the second acid (propionic acid + lactic acid), and the first acid (first acid; preferably octanoïc acid) is comprised in this composition between (about) 8 and (about) 20, preferably between (about) 9 and (about) 16, more preferably between (about) 10 and (about) 13, still more preferably between (about) 11 and (about) 12, and/or wherein the weight ratio between lactic acid and this C₆-C₁₂ fatty acid is comprised in this composition between (about) 8 and (about) 10, and/or wherein the weight ratio between lactic acid and propionic acid is comprised in this composition between (about) 1 and (about) 5, preferably between (about) 2 and (about) 4) for direct food contact sanitization; preferably, this composition is used at a temperature between (about) 0°C and (about) 25°C, more preferably between (about) 2°C and (about) 15°C, still more preferably between (about) 3°C and (about) 10°C (or even between (about) 4°C and (about) 8°C).

Another related aspect of the present invention is the use of the (diluted) composition of the present invention (preferably comprising octanoïc acid, lactic acid, propionic acid and optionally another acid, and/or wherein this (diluted) composition comprises between about 0.01 wt % and (about) 0.1 wt % (e.g. at least 0.02 wt %) of the first acid and/or wherein the weight ratio between the second acid (propionic acid + lactic acid), and the first acid (preferably octanoïc acid) is comprised in this composition between (about) 8 and (about) 20, preferably between (about) 9 and (about) 16, more preferably between (about) 10 and (about) 13, still more preferably between (about) 11 and (about) 12, and/or wherein the weight ratio between lactic acid and this first acid is comprised in this composition between (about) 8 and (about) 10, and/or wherein the weight ratio between lactic acid and propionic acid is comprised in this composition between (about) 1 and (about) 5, preferably between (about) 2 and (about) 4) for use in water treatment industry; preferably, this composition is used at a temperature between (about) 0°C and (about) 25°C, more preferably between (about) 2°C and (about) 15°C, still more preferably between (about) 3°C and (about) 10°C (or even between (about) 4°C and (about) 8°C).

Another related aspect of the present invention is the use of the (diluted) composition of the present invention (preferably comprising octanoïc acid, lactic acid, propionic acid and optionally another acid, and/or wherein this (diluted) composition comprises between about 0.005 wt % and (about) 0.1 wt % (e.g. at least 0.05 wt %) of the first acid and/or wherein the weight ratio between the second acid propionic acid + lactic acid, and the first acid (preferably octanoïc acid) is comprised in this composition between (about) 8 and (about) 20, preferably between (about) 9 and (about) 16, more preferably between (about) 10 and (about) 13, still more preferably between (about) 11 and (about) 12, and/or wherein the weight ratio between lactic acid and this first acid is comprised in this composition between (about) 8 and (about) 10, and/or wherein the weight ratio between lactic acid and propionic acid is comprised in this composition between (about) 1 and (about) 5, preferably between (about) 2 and (about) 4) for carcass disinfection; preferably, this composition is used at a temperature between (about) 0°C and (about) 25°C, more preferably between (about) 2°C and (about) 15°C, still more preferably between (about) 3°C and (about) 10°C (or even between (about) 4°C and (about) 8°C).

Another related aspect of the present invention is the use of the (diluted) composition of the present invention (preferably comprising octanoïc acid, lactic acid, propionic acid and optionally another acid, and/or wherein this (diluted) composition comprises between (about) 0.005 wt % and (about) 0.1 wt % (e.g. at least 0.05 wt %) of the first acid and/or wherein the weight ratio between the second acid (propionic acid + lactic acid, and the first acid (preferably octanoïc acid) is comprised in this composition between (about) 8 and (about) 20, preferably between (about) 9 and (about) 16, more preferably between (about) 10 and (about) 13, still more preferably between (about) 11 and (about) 12, and/or wherein the weight ratio between lactic acid and this first acid is comprised in this composition between (about) 8 and (about) 10, and/or wherein the weight ratio between lactic acid and propionic acid is comprised in this composition between (about) 1 and (about) 5, preferably between (about) 2 and (about) 4) for fruit or vegetable disinfection; preferably, this composition is used at a temperature between (about) 0°C and (about) 25°C, more preferably between (about) 2°C and (about) 15°C, still more preferably between (about) 3°C and (about) 10°C (or even between (about) 4°C and (about) 8°C).

The compositions according to the present invention successfully passed the requirements of the European standards according to EN 1276 tests and methods which is a suspension-based study formally used to evaluate anti-bacterial activity upon pathogenic test organisms (Staphylococcus aureus, Pseudomonas aeruginosa, Enterococcus hirae and Escherichia coli) the required performance criteria obtained is at least a 5 log reduction in less than 5 minutes.

### Examples

CLDI038RD168
48.0% Lactic acid 88%
14.0% acetic acid 75%
5.0% propionic acid 100%
5.0% butyl diglycol
5.2% octanoic acid
10.0% sulfuric acid 78%
12.8% city water

CLDI038RD176
50.0% Lactic acid 88%
20.0% propionic acid 100%
5.5% octanoic acid
10.0% sulfuric acid 78%
14.5% city water

CLDI038RD177
50.5% Lactic acid 88%
20.0% propionic acid 100%
5.0% octanoic acid
10.0% sulfuric acid 78%
14.5% city water

CLDI038RD178
50.5% Lactic acid 88%
20.0% propionic acid 80%
5.0% octanoic acid
10.0% sulfuric acid 78%
14.5% city water

CLDI038RD179
50.0% Lactic acid 88%
20.0% propionic acid 80%
5.5% octanoic acid
10.0% sulfuric acid 78%
14.5% city water

CLDI038RD180
55.0% Lactic acid 88%
20.0% propionic acid 80%
5.0% octanoic acid
5.0% sulfuric acid 78%
15.0% city water

CLDI038RD181
50.0% Lactic acid 88%
20.0% propionic acid 80%
4.7% octanoic acid
10.0% sulfuric acid 78%
14.3% city water

| Strains | Reference |
|---|---|
| 247 | Saccharomyces Cerevisae |
| 808 | Saccharomyces Diastaticus |
| 504 | Pediococcus Damnosus |
| 403 | Lactobacillus Brevis |

**Table 1**

| *Strains:* | *247* | *403* | *504* | *808* |
|---|---|---|---|---|
| Septacid BN 0.5% | 3.4 | 4.5 | 5.6 | 5.3 |
| Septacid BN 0.75% | 4.8 | 6.6 | 6.8 | 5.6 |
| Septacid BN 1% | 5.4 | 6.6 | 7.1 | 5.6 |
| CLDI038RD168 0.5% | 2.1 | 3.5 | 3.8 | 2.9 |
| CLDI038RD168 0.75% | 5.4 | 6.2 | 5.7 | 5.6 |
| CLDI038RD168 1% | 5.4 | 6.8 | 6.6 | 5.6 |
| CLDI038RD176 0.5% | 2.9 | 4.0 | 3.8 | 4.9 |
| CLDI038RD176 0.75% | 5.4 | 6.8 | 6.4 | 5.6 |
| CLDI038RD176 1% | 5.4 | 6.8 | 7.1 | 5.6 |
| CLDI038RD177 0.5% | 2.1 | 3.9 | 3.8 | 3.0 |
| CLDI038RD177 0.75% | 5.4 | 5.9 | 5.1 | 5.6 |
| CLDI038RD177 1% | 5.4 | 6.4 | 6.9 | 5.6 |
| CLDI038RD178 0.5% | 3.2 | 3.5 | 3.8 | 3.5 |
| CLDI038RD178 0.75% | 5.4 | 5.8 | 6.3 | 5.6 |
| CLDI038RD178 1% | 5.4 | 6.8 | 7.1 | 5.6 |
| CLDI038RD179 0.5% | 3.2 | 3.5 | 3.8 | 4.1 |
| CLDI038RD179 0.75% | 5.4 | 6.8 | 5.7 | 5.6 |
| CLDI038RD179 1% | 5.4 | 6.8 | 7.1 | 5.6 |
| CLDI038RD180 0.5% | 2.1 | 3.7 | 3.8 | 3.1 |
| CLDI038RD180 0.75% | 5.4 | 5.3 | 6.1 | 5.6 |
| CLDI038RD180 1% | 5.4 | 6.8 | 7.1 | 5.6 |
| CLDI038RD181 0.5% | 3.2 | 3.5 | 3.8 | 2.3 |
| CLDI038RD181 0.75% | 5.4 | 4.7 | 6.3 | 5.6 |
| CLDI038RD181 1% | 5.4 | 6.8 | 7.1 | 5.6 |

From these results, the inventors conclude that all these compositions can reach the 5 log reduction threshold for all the contaminants microorganisms; however, some of the compositions are more potent (at higher dilutions, and also can exert stronger sanitizing effect at lower dilutions), and are even more potent than the reference (Septacid).

Furthermore, the compositions that do not comprise enough lactic acid and/or propionic acid require the addition of emulsifiers in order to remain homogenous (e.g. at 4°C) and/or to exert antimicrobial effect, while compositions that do not comprise enough octanoïc acid cannot be used at the highest dilutions.

The inventors have further compared compositions according to the invention (comprising, beside octanoïc acid, sufficient amounts of propionic acid and/or of lactic acid) and compositions outside the present invention, comprising octanoïc acid, but without sufficient amounts of propionic acid and/or of lactic acid. The inventors have found that too low levels of these propionic acid and/or of lactic acid result into non-working compositions, unless emulsifiers are added. Furthermore, the inventors have observed that compositions comprising sufficient amounts of lactic acid and sufficient amounts of propionic acid are clearly the best ones; compositions comprising only propionic acid are quite effective, but did not allow to incorporate equal amounts of octanoïc acid, while compositions comprising only lactic acids are less potent in the cleaning conditions (loss of homogeneity, presence of a fat film).

The inventors have also tested compositions comprising strong mineral acids (sulphuric acid) and octanoïc acid. However, these compositions were inefficient and/or not stable (homogenous) at low temperature, except with the addition of sufficient amounts of propionic acid and of lactic acid or of selected artificial (non-food safe) emulsifiers.

The inventors have also tested compositions comprising organic acids (not lactic acid nor propionic acids) and octanoïc acid. However, these compositions (without lactic acid and propionic acid) were inefficient and/or not stable (homogenous) at low temperature, except with the addition of emulsifiers.

## Claims

1. A water-dilutable antimicrobial composition comprising:
a first acid being selected from the group consisting of octanoic acid, nonanoic acid and
decanoic acid or a mixture thereof, and
a second acid being a mixture of propionic acid and lactic acid,
wherein the said composition comprises at least 0.1% of the said first acid (weight first acid:weight composition), wherein the weight ratio between the said second acid and the said first acid is of at least 8 (weight second acid: weight first acid),
wherein the pH of the said composition is less than 5.0,
and wherein the said composition comprises more than 20 wt % of lactic acid (weight lactic acid: weight of the said composition) and comprises more than 20 wt % of propionic acid (weight propionic acid: weight of the said composition).

2. The water-dilutable composition according to claim 1, wherein the first acid is octanoic acid.

3. The water-dilutable composition according to the claim 1 or to the claim 2, further comprising a third acid which is a strong mineral acid selected from the group consisting of nitric acid, phosphoric acid and sulphuric acid.

4. The water-dilutable composition according to the claim 3, which comprises between 1 wt % and 20 wt % of the strong mineral acid (weight strong mineral acid: weight of the said composition).

5. The water-dilutable composition according to claim 1 or claim 2, further comprising a third acid, which is selected from the group consisting of acetic acid, citric acid, tartaric acid, and mixture thereof.

6. The water-dilutable composition according to claim 5, which comprises between 1 wt% and 20 wt% of acetic acid and/or citric acid and/or tartaric acid (weight acid: weight of the said composition).

7. The water-dilutable composition according to any one of the preceding claims, wherein the first acid, the second acid and possibly the third acid are present in an amount comprised between 50% and 99% (sum of the weight of all the said acids: weight of the said composition).

8. The water-dilutable composition according to any one of the preceding claims, comprising more than 1% of the first acid (weight of the first acid: weight of the composition.

9. The water dilutable composition according to any one of the preceding claims, comprising more than 5 % of the first acid (weight of the first acid: weight composition).

10. The water-dilutable composition according to any one of the preceding claims being detergent-free.

11. A non-therapeutic use of a composition according to any one of the preceding claims 1 to 10 for sanitizing a surface.

12. A non-therapeutic method for sanitizing a surface, comprising the step of adding to the said surface, a composition comprising a first acid being selected from the group consisting of octanoic acid, nonanoic acid and decanoic acid or a mixture thereof, and a second acid being a mixture of propionic acid and lactic acid, wherein the said composition comprises at least 0.1 % of the said first acid (weight first acid: weight composition), wherein the weight ratio between the said second acid and the said first acid is of at least 8, wherein the pH of the said composition is less than 5.0, and wherein the said composition comprises more than 20 wt % of propionic acid (weight propionic acid: weight of the said composition), and the said second acid comprises at least 20 wt % of lactic acid (weight lactic acid: weight composition).

13. The method according to the claim 12, wherein the composition comprising the first acid and the second acid is added at a temperature comprised between 2°C and 25°C.

14. The method according to the claim 12 or 13, not comprising water rinsing and/or washing step after the addition of the composition comprising the first acid and the second acid.

15. The method according to any one of the preceding claims, wherein the surface is a bottle surface.

## Patentansprüche

1. Eine wasserverdünnbare antimikrobielle Zusammensetzung, umfassend:
eine erste Säure, die aus der Gruppe ausgewählt ist, die aus Octansäure, Nonansäure und Decansäure oder einer Mischung davon besteht, und
eine zweite Säure, die eine Mischung von Propionsäure und Milchsäure ist,
wobei die besagte Zusammensetzung zumindest 0,1 % der ersten Säure (Gewicht erste Säure:Gewicht Zusammensetzung) umfasst, wobei das Gewichtsverhältnis zwischen der besagten zweiten Säure und der besagten ersten Säure zumindest 8 (Gewicht zweite Säure:Gewicht erste Säure) beträgt,
wobei der pH-Wert der besagten Zusammensetzung weniger als 5,0 beträgt,
und wobei die Zusammensetzung mehr als 20 Gew.-% Milchsäure (Gewicht Milchsäure:Gewicht der besagten Zusammensetzung) umfasst und mehr als 20 Gew.-% Propionsäure (Gewicht Propionsäure:Gewicht der besagten Zusammensetzung) umfasst.

2. Die wasserverdünnbare Zusammensetzung nach Anspruch 1, wobei die erste Säure Octansäure ist.

3. Die wasserverdünnbare Zusammensetzung nach Anspruch 1 oder Anspruch 2, die ferner eine dritte Säure umfasst, die eine starke Mineralsäure ist, die aus der Gruppe ausgewählt ist, die aus Salpetersäure, Phosphorsäure und Schwefelsäure besteht.

4. Die wasserverdünnbare Zusammensetzung nach Anspruch 3, die zwischen 1 Gew.-% und 20 Gew.-% der starken Mineralsäure (Gewicht starke Mineralsäure:Gewicht der Zusammensetzung) umfasst.

5. Die wasserverdünnbare Zusammensetzung nach Anspruch 1 oder Anspruch 2, die ferner eine dritte Säure umfasst, die aus der Gruppe ausgewählt ist, die aus Essigsäure, Zitronensäure, Weinsäure und deren Mischung besteht.

6. Die wasserverdünnbare Zusammensetzung nach Anspruch 5, die zwischen 1 Gew.-% und 20 Gew.-% Essigsäure und/oder Zitronensäure und/oder Weinsäure (Gewicht Säure:Gewicht der besagten Zusammensetzung) umfasst.

7. Die wasserverdünnbare Zusammensetzung nach irgendeinem der vorstehenden Ansprüche, wobei die erste Säure, die zweite Säure und gegebenenfalls die dritte Säure in einer Menge vorhanden sind, die zwischen 50 % und 99 % (Summe des Gewichts aller besagten Säuren:Gewicht der besagten Zusammensetzung) beträgt.

8. Die wasserverdünnbare Zusammensetzung nach irgendeinem der vorstehenden Ansprüche, die die erste Säure zu mehr als 1 % (Gewicht der ersten Säure:Gewicht der Zusammensetzung) umfasst.

9. Die wasserverdünnbare Zusammensetzung nach irgendeinem der vorstehenden Ansprüche, die die erste Säure zu mehr als 5 % (Gewicht der ersten Säure:Gewicht Zusammensetzung) umfasst.

10. Die wasserverdünnbare Zusammensetzung nach irgendeinem der vorstehenden Ansprüche, die detergensfrei ist.

11. Eine nichttherapeutische Anwendung einer Zusammensetzung nach irgendeinem der vorstehenden Ansprüche 1 bis 10 zum hygienischen Reinigen einer Oberfläche.

12. Ein nichttherapeutisches Verfahren zum hygienischen Reinigen einer Oberfläche, das den Schritt des Hinzufügens einer Zusammensetzung, die eine erste Säure, ausgewählt aus der Gruppe, die aus Octansäure, Nonansäure und Decansäure oder einer Mischung davon besteht, und eine zweite Säure, die eine Mischung von Propionsäure und Milchsäure ist, auf die besagte Oberfläche umfasst wobei die besagte Zusammensetzung die erste Säure zu zumindest 0,1 % (Gewicht erste Säure:Gewicht Zusammensetzung) umfasst, wobei das Gewichtsverhältnis zwischen der besagten zweiten Säure und der besagten ersten Säure zumindest 8 beträgt, wobei der pH-Wert der besagten Zusammensetzung weniger als 5,0 beträgt, und wobei die besagte Zusammensetzung mehr als 20 Gew.-% Propionsäure (Gewicht Propionsäure:Gewicht der besagten Zusammensetzung) umfasst und die besagte zweite Säure zumindest 20 Gew.-% Milchsäure (Gewicht Milchsäure:Gewicht der Zusammensetzung) umfasst.

13. Das Verfahren nach Anspruch 12, wobei die Zusammensetzung, die die erste Säure und die zweite Säure umfasst, bei einer Temperatur zwischen 2 °C und 25 °C hinzugefügt wird.

14. Das Verfahren nach Anspruch 12 oder 13, das keinen Wasserspül- und/oder Waschschritt nach dem Hinzufügen der Zusammensetzung, umfassend die erste Säure und die zweite Säure, umfasst.

15. Das Verfahren nach irgendeinem der vorstehenden Ansprüche, wobei die Oberfläche eine Flaschenoberfläche ist.

## Revendications

1. Composition antimicrobienne diluable dans l'eau comprenant :
un premier acide choisi parmi le groupe constitué par l'acide octanoïque, l'acide nonanoïque et l'acide décanoïque ou un mélange de ceux-ci, et
un deuxième acide qui est un mélange d'acide propionique et d'acide lactique,
dans laquelle ladite composition comprend au moins 0,1 % dudit premier acide (poids du premier acide:poids de la composition), dans laquelle le rapport pondéral entre ledit deuxième acide et ledit premier acide est d'au moins 8 (poids du deuxième acide:poids du premier acide ),
dans laquelle le pH de ladite composition est inférieur à 5,0,
et dans laquelle ladite composition comprend plus de 20 % en poids d'acide lactique (poids d'acide lactique:poids de ladite composition) et comprend plus de 20 % en poids d'acide propionique (poids d'acide propionique- poids de ladite composition).

2. Composition diluable dans l'eau selon la revendication 1, dans laquelle le premier acide est l'acide octanoïque.

3. Composition diluable dans l'eau selon la revendication 1 ou la revendication 2, comprenant en outre un troisième acide qui est un acide minéral fort choisi parmi le groupe constitué par l'acide nitrique, l'acide phosphorique et l'acide sulfurique.

4. Composition diluable dans l'eau selon la revendication 3, qui comprend entre 1 % en poids et 20 % en poids de l'acide minéral fort (poids d'acide minéral fort:poids de ladite composition).

5. Composition diluable dans l'eau selon la revendication 1 ou la revendication 2, comprenant en outre un troisième acide, qui est choisi parmi le groupe constitué par l'acide acétique, l'acide citrique, l'acide tartrique et un mélange de ceux-ci.

6. Composition diluable dans l'eau selon la revendication 5, qui comprend entre 1 % en poids et 20 % en poids d'acide acétique et/ou d'acide citrique et/ou d'acide tartrique (poids d'acide-poids de ladite composition).

7. Composition diluable dans l'eau selon l'une quelconque des revendications précédentes, dans laquelle le premier acide, le deuxième acide et éventuellement le troisième acide sont présents en une quantité comprise entre 50 % et 99 % (somme du poids de tous lesdits acides: poids de ladite composition).

8. Composition diluable dans l'eau selon l'une quelconque des revendications précédentes, comprenant plus de 1 % du premier acide (poids du premier acide:poids de la composition).

9. Composition diluable dans l'eau selon l'une quelconque des revendications précédentes, comprenant plus de 5 % du premier acide (poids du premier acide:poids de la composition).

10. Composition diluable dans l'eau selon l'une quelconque des revendications précédentes qui est exempte de détergent.

11. Utilisation non thérapeutique d'une composition selon l'une quelconque des revendications précédentes 1 à 10 pour assainir une surface.

12. Méthode non thérapeutique d'assainissement d'une surface, comprenant l'étape d'ajout sur ladite surface, d'une composition comprenant un premier acide choisi parmi le groupe constitué par l'acide octanoïque, l'acide nonanoïque et l'acide décanoïque, ou un mélange de ceux-ci, et un deuxième acide qui est un mélange d'acide propionique et d'acide lactique, dans laquelle ladite composition comprend au moins 0,1 % dudit premier acide (poids du premier acide:poids de la composition), dans laquelle le rapport pondéral entre ledit deuxième acide et ledit premier acide est d'au moins 8, dans laquelle le pH de ladite composition est inférieur à 5,0, et dans laquelle ladite composition comprend plus de 20 % en poids d'acide propionique (poids d'acide propionique- poids de ladite composition) et ledit deuxième acide comprend au moins 20 % en poids d'acide lactique (poids d'acide lactique:poids de la composition).

13. Méthode selon la revendication 12, dans laquelle la composition comprenant le premier acide et le deuxième acide est ajoutée à une température comprise entre 2 °C et 25 °C.

14. Méthode selon la revendication 12 ou 13, ne comprenant pas d'étape de rinçage et/ou de lavage à l'eau après l'ajout de la composition comprenant le premier acide et le deuxième acide.

15. Méthode selon l'une quelconque des revendications précédentes, dans laquelle la surface est une surface de bouteille.
